# EUROPEAN PATENT APPLICATION

(11) **EP 4 834 986 A1**
(43) Date of publication of application: **04.11.2026**
(21) Application number: 24910836.6
(22) Date of filing: 17.12.2024
(51) Int. Cl.: C08L 5/08, C08J 3/075, A61L 27/52, A61L 27/20

(54) **INJECTABLE INTERLOCKING CROSS-LINKED HYDROGEL OF HYALURONAN OR SALT THEREOF, PREPARATION METHOD THEREFOR AND USE THEREOF**

(30) Priority: 29.12.2023 CN 202311844532
(71) Applicant: Imeik Technology Development Co., Ltd., Beijing 100022 (CN)
(72) Inventor: HUANG, Fei, Beijing 100022 (CN); CAO, Jiahua, Beijing 100022 (CN); LI, Ruizhi, Beijing 100022 (CN); ZHANG, Kun, Beijing 100022 (CN)
(74) Representative: Novagraaf Technologies
(86) International application number: PCT/CN2024/139963
(87) International publication number: WO 2025/139905

(57) **Abstract**

An injectable interlocking cross-linked hyaluronan or a salt thereof, a preparation method and the use thereof. The preparation process of the hydrogel comprises a two-step cross-linking step of the hyaluronan or salt thereof with a crosslinking agent, such as an endogenous polyamine, and a dialysis step. In the two-step cross-linking, the first cross-linking step forms a network structure, and the second step cross-linking further forms an interlocking structure. The prepared hydrogel is subjected to moist heat sterilization. The prepared hydrogel has the advantages of a strong anti-degradation ability, excellent viscosity, good supporting, easy injection, high safety, and easy reconstitution, and thus meets the clinical requirements for injectable products. Moreover, the whole process is simple, convenient and rapid to operate, and is also easy to industrialize.

## Description

### Technical Field

The present invention relates to the field of biomedical materials, and in particular, to an injectable interlocking cross-linked hydrogel of hyaluronan or salt thereof, a preparation method therefor, and use thereof.

### Background

Hyaluronan (HA) and its salts are macromolecular polysaccharide compounds with high biocompatibility, and are widely distributed in the extracellular matrix of connective tissues of animals and humans, and have achieved commercialization and application in the fields of medicine and cosmetics. A large amount of hyaluronan is distributed in the skin tissue of a human body, and the hyaluronan plays a role of retaining water, maintaining an extracellular space, promoting cell repair, adhering an inner interface of the skin tissue, lubricating, etc., and therefore the hyaluronan is often used as a filling matrix for treating facial rejuvenation and contour restoration. However, the injection of unmodified hyaluronan into skin tissue has the disadvantages of poor mechanical performance, low viscosity, short residence time in vivo, etc.; moreover, optimizing the performance thereof by means of a chemical method is a common strategy for sodium hyaluronate applications.

Most commercially available hyaluronan fillers use BDDE as a cross-linking agent, and it is well known that very low concentrations of BDDE residues may be genetically toxic. Given the BDDE-associated safety risks, commercially available dermal filler products have an annual dose limited to no more than 20 mL (e.g. Juvéderm^{®}), a maximum dose per use limited to 6 mL (e.g. Restylane^{®}). A more safer and effective crosslinker is therefore an urgent need for the future development of hyaluronan based fillers.

Patent CN113499480 reports a physical and chemical dual network hydrogel for a subcutaneous filling agent and a preparation method and use thereof. In the patent, firstly, HA is chemically modified via cross-linked, and then physical freeze-thaw is used to achieve secondary cross-linking of the chemically cross-linked gel. Although the molecular lattice of a polymeric material changes during freeze-thaw, physical cross-linking is still not as stable as chemical cross-linking. Patent CN113896915 reports the preparation of cross-linked sodium hyaluronate using endogenous polyamines, including spermine and/or spermidine, as novel crosslinkers, however, the gel prepared by the process has a relatively low viscosity and a relatively high extrusion force, and at the same time, under the cross-linking system, a relatively large number of ester linkages will be formed, and when the concentration of sodium hyaluronate is relatively low (e.g. less than 15 mg/mL), the "water-gel separation" tends to occur, therefore, its clinical application as an injectable filling product is rather limited.

### Summary

In view of the disadvantages of the prior art, an object of the present invention is to provide an injectable interlocking cross-linked hydrogel of hyaluronan or salt thereof, a preparation method therefor, and use thereof.

The present invention provides an injectable interlocking cross-linked hydrogel of hyaluronan or salt thereof, which uses a double cross-linked interlock technology (DCIT) to form an interlocking network structure in a two-step chemical cross-linking reaction process. First, the hyaluronan or salt thereof and the endogenous polyamine as the cross-linking agent are cross-linked in a first step to form a gel with a network structure, and the mixture is then cross-linked in a second step to form a hydrogel having an interlocking structure.

The present invention is achieved by the following technical solutions.

In a first aspect, the invention provides an injectable interlocking cross-linked hydrogel of hyaluronan or salt thereof, the hydrogel is obtained by a two-step cross-linking reaction, firstly performing a first step cross-linking reaction on the hyaluronan or salt thereof and a cross-linking agent to form a network structure, and subsequently interpenetrating the hyaluronan or salt thereof and the cross-linking agent into the network structure, performing a second step cross-linking reaction to obtain an injectable interlocking cross-linked hydrogel of hyaluronan or salt thereof.

The hyaluronan or salt thereof is selected from one or more of sodium hyaluronate, zinc hyaluronate, potassium hyaluronate and calcium hyaluronate, preferably sodium hyaluronate.

The hyaluronan or salt thereof has a molecular weight of 500-2600KDa.

The cross-linking agent is an endogenous polyamine, including spermidine, spermine and derivatives thereof.

The particle size D50 of the hydrogel of hyaluronan or salt thereof is 50-650 µm.

The concentration of the hyaluronan or salt thereof in the injectable interlocking cross-linked hydrogel of hyaluronan or salt thereof is 10-25 mg/mL.

In the first step cross-linking reaction, the molar ratio of the cross-linking agent to the hyaluronan or salt thereof is 0.01-0.1, and in the second step cross-linking reaction, the molar ratio of the cross-linking agent to the hyaluronan or salt thereof is 0.01-0.1.

In the two-step cross-linking, the first step cross-linking forms a network structure of the hyaluronan or a salt thereof, and the second step cross-linking further forms an interlocking structure, thereby transitioning the structure from interpenetration to interlock.

Wherein the molar amount of hyaluronan or salt thereof = the mass of hyaluronan or salt thereof used/the molecular weight of the disaccharide repeating unit of hyaluronan or salt thereof (403 g/mol);
Preferably, the cross-linking process requires the addition of a catalyst selected from one or more of a carbodiimide, a bromophosphonium salt formed from triphenylphosphine and a bromide, a carbonium salt, and 4-(4,6-dimethoxytriazin-2-yl)-4-methylmorpholine hydrochloride (DMTMM).

In the first step cross-linking reaction, the molar ratio of the catalyst to the hyaluronan or salt thereof is 0.6-3, and in the second step cross-linking reaction, the molar ratio of the catalyst to the hyaluronan or salt thereof is 0.03-0.3.

Further, after the two-step cross-linking reaction, a dialysis step is further preformed to obtain the injectable interlocking cross-linked hydrogel of hyaluronan or salt thereof.

Preferably, the dialysis is carried out first with a alkaline dialysate and then with a neutral dialysate.

The interlocking cross-linked hydrogel of hyaluronan or salt thereof requires dialysis under a suitable alkaline condition to remove unstable ester bonds, and the injectable interlocking cross-linked hydrogel of hyaluronan or salt thereof is finally obtained. It has the advantages of strong anti-degradation ability, excellent viscosity, good support, ease of extrusion, high safety and the like, and can be used for subcutaneous filling by injection.

In a second aspect, the present invention provides a method for preparing the injectable interlocking cross-linked hydrogel of hyaluronan or salt thereof of the first aspect, and the specific steps are as follows:
(1) mixing a cross-linking agent with hyaluronan or salt thereof to prepare a mixed solution A of the cross-linking agent and the hyaluronan or salt thereof;
(2) adding a catalyst to the mixed solution A of the cross-linking agent and hyaluronan or salt thereof, and performing a first step cross-linking reaction, so as to prepare a cross-linked gel having a network structure;
(3) mixing the cross-linking agent with the hyaluronan or salt thereof to prepare a mixed solution B of the cross-linking agent and the hyaluronan or salt thereof;
(4) adding the mixed solution B prepared in step (3) to the cross-linked gel having a network structure prepared in step (2), performing a second cross-linking reaction in the presence of a catalyst to prepare a cross-linked gel having an interlocking structure, and performing dialysis.

The interlocking cross-linked hydrogel of hyaluronan or salt thereof of the present invention uses double cross-linked interlock technology (DCIT). During the first step cross-linking, carboxyl groups in molecules of the hyaluronan or salt thereof (HA) and multiple amino sites of spermine or spermidine can form amide bonds in the presence of a catalyst to form a network structure; meanwhile, the carboxyl group in the HA molecule and the hydroxyl group activated by the catalyst can form an ester bond, which has a occupancy effect; then an HA solution, a cross-linking agent and a catalyst are added, and a second step cross-linking reaction is performed; in this process, the HA molecules and the cross-linking agent can interpenetrate evenly into the network structure formed in the first step with stirring, and then form amide bonds in the presence of the catalyst to form an interlocking structure.

Preferably, the cross-linking agents in steps (1) and (3) are endogenous polyamines, and further preferably include spermidine, spermine and derivatives thereof.

Preferably, in steps (1) and (3), the cross-linking agent is first dissolved in water, and the pH is adjusted to 6.0-6.3.

The hyaluronan or salt thereof in steps (1) and (3) is selected from one or more of sodium hyaluronate, zinc hyaluronate, potassium hyaluronate and calcium hyaluronate, preferably sodium hyaluronate.

Further, the hyaluronan or salt thereof has a molecular weight of 500-2600KDa.

Preferably, the mixing and dissolution time in steps (1), and (3) is 3-5 h, for example: 3 h, 3.5 h, 4 h, 4.5 h, 5 h.

In the mixed solution A and the mixed solution B, the mass percentage of the hyaluronan or salt thereof is 1%-5%.

Preferably, in the mixed solution A and the mixed solution B, the molar ratio of the cross-linking agent to the hyaluronan or salt thereof in each is 0.01 to 0.1, and the amounts of the cross-linking agents in the two solutions may be the same or different, for example, 0.01, 0.02, 0.03, 0.04, 0.05, 0.06, 0.07, 0.08, 0.09 and 0.1.

More preferably, the molar ratio of the cross-linking agent to the hyaluronan or salt thereof in step (1) is less than the molar ratio of the cross-linking agent to the hyaluronan or salt thereof in step (3), that is, the degree of cross-linking in the first step reaction is lower than that in the second step reaction, because the first step cross-linking reaction produces a relatively loose first network structure, so that the hyaluronan or salt thereof (HA) molecule added in the second step can be easily interpenetrated into the network structure, and a space structure with different layers is constructed, which further facilitates the formation of an interlocking structure.

Preferably, the cross-linking process requires a catalyst, and the catalyst in steps (2) and (4) is selected from one or more of a brominated phosphonium salt, a carbonium salt formed from a carbodiimide, a bromophosphonium salt formed from triphenylphosphine and a bromide, a carbonium salt, and 4-(4,6-dimethoxytriazin-2-yl)-4-methylmorpholine hydrochloride (DMTMM), and the two may be the same or different.

Preferably, the catalyst in steps (2) and (4) is 1-ethyl-3-(3-dimethylaminopropyl) carbodiimide (EDC), and an auxiliary agent is added at the same time to improve the efficiency of the cross-linking reaction.

Preferably, the auxiliary agent is selected from any one or more of N-hydroxysuccinimide (NHS), sulfonated N-hydroxysuccinimide (Sulfo-NHS), tert-butanol, 1-hydroxybenzotriazole (HOBt), and is further preferably NHS. The molar ratio of the auxiliary agent to the catalyst is 0.2-0.4.

Preferably, in step (2), the molar ratio of the catalyst to the hyaluronan or salt thereof in the mixed solution A is 0.6 -3, for example, 0.6, 1, 1.5, 2, 2.5, 3, and more preferably 1-2.

The cross-linking reaction time in step (2) is 1-3 h, for example: 1 h, 2 h, 3 h.

In step (2), in the first step cross-linking reaction, a larger amount of catalyst can be added to form more ester bonds, which exerts a occupancy effect, and is beneficial to improving the cross-linking efficiency of the second step cross-linking reaction, and at the same time reducing the amount of catalyst used in the second step cross-linking reaction, so that the cross-linking reaction forms more interlocking structures under the reaction conditions of a low dose of catalyst and a safe cross-linking agent, so as to improve the degradation resistance of the final product.

Preferably, in step (4), the molar ratio of the catalyst to the hyaluronan or salt thereof in the mixed solution B is 0.03-0.3, e.g., 0.03, 0.05, 0.1, 0.15, 0.2, 0.25, 0.3.

The cross-linking reaction time in step (4) is 1-24 h, for example: 1 h, 2 h, 3 h, 8 h, 12 h, 16 h, 20 h, 24 h.

Preferably, the dialysis in step (4) is a two-step dialysis, wherein the first step is an alkaline dialysis and the second step is a neutral dialysis.

In order to sufficiently form the interlocking structure gel, it is necessary to form a pre-occupied ester bond structure (including an EDC active ester, an NHS active ester, an ester of an HA hydroxyl group with a carboxyl group, etc.) in a first step cross-linking reaction, however, the substance having an ester bond structure has instability, and at the same time affects the stability of a gel product. The present invention employs subsequent alkaline dialysis to open the pre-formed ester bonds during alkaline dialysis, thereby releasing linear HA chains. These HA chains not only serve as a lubricant to improve the injectability of the product, but can also further intertwine with or interpenetrate into the formed interlocking structure, thereby enhancing intermolecular interactions and improving the viscosity of the product.

Preferably, the molar ratio of hydroxyl in the alkaline dialysate to the total hyaluronan or salt thereof used in steps (1) and (3) (total amount in mixed solutions A and B) is 1:1-10:1, such as: 1:1, 5:1, 10:1.

The solute of the alkaline dialysate is selected from sodium hydroxide and potassium hydroxide, and the solvent of the alkaline dialysate is a combination of any one or more of purified water, normal saline or phosphate buffer.

The unstable ester bond structure produced in the two-step cross-linking reaction can be destroyed alkaline dialysis, thereby improving the resistance to moist heat sterilization and storage stability of the final gel product; at the same time, alkaline conditions will also destroy the glycosidic bonds of HA to a certain extent, and therefore it is necessary to control the molar ratio of hydroxide to hyaluronan or salt thereof in the alkaline dialysis solution, and if the molar amount of hydroxide is relatively low, the unstable ester bond structure material cannot be effectively removed, and the stability of the hydrogel product cannot be ensured; if the molar amount of hydroxide is high, the glycosidic bonds of HA will be destroyed, the network structure of cross-linked HA is not stable enough and can be easily degraded, or the concentration of HA in a product decreases too quickly, which cannot meet clinical injection requirements.

Further preferably, in the alkaline dialysis, the volume ratio of the the dialysate to the cross-linked having an interlocking structure is 15:1-25:1, and the dialysis temperature is 10-40°C, for example, 10°C, 15°C, 20°C, 25°C, 30°C, 35°C and 40°C; the dialysis time is 1-10 h, for example: 1 h, 2 h, 4 h, 6 h, 8 h, 10 h.

Preferably, in the neutral dialysis, the dialysate comprises a combination of any one or more of purified water, normal saline and phosphate buffer, and the pH of the neutral dialysate can be 6.0-7.6.

Further preferably, in the neutral dialysis, the volume ratio of the dialysis solution to the cross-linked gel having an interlocking structure is 15:1-30:1, for example, 15:1, 17:1, 20:1, 23:1, 25:1, 27:1, 30:1; the dialysis temperature is 10-40°C and the dialysis time is 1-20 h.

Prior to the alkaline dialysis treatment, the cross-linked gel having an interlocking structure obtained from the second step cross-linking reaction is cut into pieces with an equivalent diameter of 0.02-1cm, for example: 0.02 cm, 0.05 cm, 0.1 cm, 0.15 cm, 0.2 cm, 0.25 cm, 0.3 cm, 0.35 cm, 0.4 cm, 0.45 cm, 0.5 cm, 0.55 cm, 0.6 cm, 0.65 cm, 0.7 cm, 0.75 cm, 0.8 cm, 0.85 cm, 0.9 cm, 0.95 cm, 1 cm.

Preferably, the molecular weight cut-off (MWCO) of the dialysis bag is 1-100 KDa e.g.: 1 KDa, 5 KDa, 10 KDa, 20 KDa, 30 KDa, 40 KDa, 50 KDa, 60 KDa, 70 KDa, 80 KDa, 90 KDa, 100 KDa.

Optionally, the preparation method comprises step (5): performing dilution, pulverization, filling, and moist heat sterilization after dialysis to obtain the injectable interlocking cross-linked hydrogel of hyaluronan or salt thereof.

Preferably, the diluted solution of the cross-linked gel having an interlocking structure in step (5) is selected from one or more of purified water, normal saline, phosphate balanced solution, or phosphate buffered solution. When purified water is used, the osmolality of the system needs to be adjusted in the range of 200 mOsmol/kg-400 mOsmol/kg using sodium dihydrogen phosphate and disodium hydrogen phosphate, and the pH value is adjusted to 6.0-7.6.

Preferably, in step (5), lidocaine hydrochloride is added to the cross-linked gel having an interlocking structure after dilution, and the gel is crushed. The addition of lidocaine hydrochloride can reduce pain upon injection of the hydrogel of hyaluronan or salt thereof.

Preferably, the lidocaine HCl concentration is 1-5 mg/mL, for example: 1 mg/mL, 2 mg/mL, 3 mg/mL, 4 mg/mL, 5 mg/mL.

Preferably, the gel particle size D50 after grinding in step (5) is 50-450 µm, and D90 is 100 -650 µm.

Preferably, the moist heat sterilization parameters in step (5) are 116-121°C, 15-30 min.

Preferably, in step (5), in the injectable interlocking cross-linked hydrogel of hyaluronan or salt thereof, the concentration of hyaluronan or salt thereof is 10 -25 mg/mL, for example, 10 mg/mL, 15 mg/mL, 16 mg/mL, 17 mg/mL, 18 mg/mL, 19 mg/mL, 20 mg/mL, 21 mg/mL, 23 mg/mL, 25 mg/mL.

In a third aspect, the present invention provides an application of the injectable interlocking cross-linked hydrogel of hyaluronan or salt thereof according to the first aspect or the injectable interlocking cross-linked hydrogel of hyaluronan or salt thereof obtained according to the preparation method of the second aspect in preparing a non-therapeutic tissue filling and repairing material or a pharmaceutical carrier.

In particular, the tissue filling can be used to eliminate wrinkles (e.g., periocular wrinkles, frontal folds, glabellar wrinkles, perioral wrinkles, nasolabial folds, tear trough, marionette lines, cervical, hand wrinkles, stretch marks, etc.), anti-aging, elimination of scars, wound repair, intraoperative and post-operative venous hemostasis, etc.

The tissue repair material may be a bone tissue repair material, a cartilage tissue repair material, a corneal tissue repair material, a cardiovascular tissue repair material, a liver tissue repair material, and the like.

The drug uses an interlocking cross-linked hydrogel of hyaluronan or salt thereof as a carrier, so as to achieve the purposes of slow release, controlled release, targeted drug delivery and the like.

Beneficial effects of the present invention:
I. The interlocking cross-linked hydrogel of the present invention uses an endogenous polyamine as a cross-linking agent, and compared with existing gels, the gel of the present invention has higher safety.
II. The cross-linked hydrogel with an interlocking structure of the present invention is subjected to a double cross-linked interlock technology (DCIT) to sequentially undergo two cross-linking reactions, and by controlling the amounts of a cross-linking agent and a catalyst in the first step cross-linking reaction, the ester bond occupancy effect is fully utilized in the first step cross-linking reaction, the efficiency of the second step cross-linking reaction is improved, and upgrading from an interpenetrating network to an interlocking structure is achieved; furthermore, by means of an alkaline dialysis process, the originally occupied ester bonds in a cross-linking reaction process is destroyed, and the released HA chains not only increases the injectability of a product, but also further intertwines with an interlocking structure, thereby enhancing intermolecular interactions. The prepared gel exhibits high supporting properties and viscosity as well as an excellent enzymatic degradation resistance, and satisfies clinical requirements for an injectable product.
III. The cross-linked hydrogel with an interlocking structure of the present invention uses a highly efficient alkaline dialysis process, and can remove processing aids in a short time, thereby ensuring that the concentration of the hyaluronan or salt thereof in the dialyzed gel is higher than the concentration of the hyaluronan or salt thereof in an expected product. The subsequent concentration procedure is omitted, the production efficiency is improved, and damage to the polymer crystal lattice and the phenomena of being difficult in redissolution during concentration are avoided.

### Brief Description of the Drawings

Fig. 1 is a schematic diagram of a gel cross-linking reaction process of the present invention;
Fig. 2 shows the appearance of a gel product of the present invention, where A represents the sample of Example 2 and B represents the sample of Comparative Example 1.

### Detailed Description of the Embodiments

The technical solutions of the present invention are described in detail below with reference to the embodiments and the accompanying drawings. The embodiments described are only a part rather than all of the embodiments of the present invention. Other embodiments obtained by one of ordinary skill in the art based on the embodiments of the present invention without creative efforts fall within the scope of protection of the present invention.

It should be noted that, unless otherwise specified, the experimental methods and reagents used in the embodiments of the present invention are conventional experimental methods and reagents in the art.

### Example 1 Preparation of an injectable interlocking cross-linked sodium hyaluronate hydrogel

(1) An 8% aqueous solution of spermidine having a pH = 6.0 was prepared and stored refrigerated for use. 0.9 g of the solution was weighed and dispersed into 250 mL of purified water, 5 g of sodium hyaluronate powder was added thereto (the molar ratio of spermidine to sodium hyaluronate was 0.04, and the concentration of HA was 20 mg/mL), stirred until the powder was completely dissolve, and the dissolution time was 3 h.
(2) Then, 2.37 g EDC (the molar ratio of EDC to sodium hyaluronate was 1.00) and 0.48 g NHS were weighed and added into the aforementioned mixed solution, stirred at room temperature for 40 min, and then the system was placed in a 40°C environment for a cross-linking reaction for 3 h, so as to prepare a network cross-linked gel.
(3) 0.9 g of the spermidine solution was weighed and dispersed into 250 mL of purified water, 5 g of sodium hyaluronate powder was added thereto (the molar ratio of spermidine to sodium hyaluronate was 0.04, the concentration of HA was 20 mg/mL), stirred until the powder was completely dissolved, and the dissolution time was 3 h.
(4) The mixed solution of step (3) was added to the network cross-linked gel of step (2), stirred for 10 min, and then crushed for 3 min with a homogenizer at 10000 rpm with 5 min intervals, and repeated 5 times. Then, 0.284 g EDC (the molar ratio of EDC to sodium hyaluronate was 0.12) and 0.057 g NHS were added into the aforementioned system, and the system was further stirred at room temperature for 40 min, and then the system was placed in a 40°C environment for a cross-linking reaction for 16 h to prepare an interlocking cross-linked gel.

The interlocking cross-linked gel was cut with scissors into small pieces of 0.5 cm equivalent diameter and filled into dialysis bags, the dialysis bag was sealed at both ends with a clamp and placed in 9 L phosphate solution containing 2 g sodium hydroxide (the molar ratio of hydroxide to sodium hyaluronate was 2) for dialysis, the dialysis temperature was 40°C, and after 2 h, the solution was changed into a 9 L neutral phosphate solution for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(5) The gel having a concentration C was diluted and compounded until the concentration of sodium hyaluronate was 15 mg/mL, lidocaine hydrochloride was added, using a neutral dialysate as a diluent, and the content of lidocaine hydrochloride in the gel was 3 mg/mL. The compounded gel was crushed, filled into a pre-filled syringe, and subjected to moist heat sterilization at 121°C for 15 min to obtain an injectable interlocking cross-linked sodium hyaluronate hydrogel.

### Example 2 Preparation of an injectable interlocking cross-linked sodium hyaluronate hydrogel

(1) An 8% aqueous solution of spermidine having a pH = 6.0 was prepared and stored refrigerated for use. 0.68 g of the solution was weighed and dispersed into 250 mL of purified water, 5 g of sodium hyaluronate powder was added thereto (the molar ratio of spermidine to sodium hyaluronate was 0.03, the concentration of HA was 20 mg/mL), stirred until the powder was completely dissolved, and the dissolution time was 3 h.
(2) 2.37 g of EDC (the molar ratio of EDC to sodium hyaluronate was 1.00) and 0.48 g of NHS were weighed and added into the aforementioned mixed solution, stirred at room temperature for 40 min, and then the system was placed in a 40°C environment for a cross-linking reaction for 3 h, so as to prepare a network cross-linked gel.
(3) 1.13 g of the spermidine solution was weighed and dispersed into 250 mL of purified water, 5 g of sodium hyaluronate powder was added thereto (the molar ratio of spermidine to sodium hyaluronate was 0.05, the concentration of HA was 20 mg/mL), stirred until the powder was completely dissolved, and the dissolution time was 3 h.
(4) The mixed solution of step (3) was added to the network cross-linked gel of step (2), stirred for 10 min, and then crushed for 3 min with a homogenizer at 10000 rpm with 5 min intervals, and repeated 5 times. Then, 0.355 g EDC (the molar ratio of EDC to sodium hyaluronate was 0.15) and 0.071 g NHS were added into the aforementioned system, and the system was further stirred at room temperature for 40 min, and then the system was placed in a 40°C environment for a cross-linking reaction for 16 h to prepare an interlocking cross-linked gel.

The interlocking cross-linked gel was cut with scissors into small pieces of 0.5 cm equivalent diameter and filled into dialysis bags, the dialysis bag was sealed at both ends with a clamp and placed in 9 L phosphate solution containing 2 g sodium hydroxide (the molar ratio of hydroxide to sodium hyaluronate was 2) for dialysis, the dialysis temperature was 40°C, and after 2 h, the solution was changed into a 9 L neutral phosphate solution for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(5) The gel having a concentration C was diluted and compounded until the concentration of sodium hyaluronate was 15 mg/mL, lidocaine hydrochloride was added, using a neutral dialysate as a diluent, and the content of lidocaine hydrochloride in the gel was 3 mg/mL. The compounded gel was crushed, filled into a pre-filled syringe, and subjected to moist heat sterilization at 121°C for 15 min to obtain an injectable interlocking cross-linked sodium hyaluronate hydrogel.

### Example 3 Preparation of an injectable interlocking cross-linked sodium hyaluronate hydrogel

(1) An 8% aqueous solution of spermidine having a pH = 6.0 was prepared and stored refrigerated for use. 1.13 g of the solution was weighed and dispersed into 250 mL of purified water, 5 g of sodium hyaluronate powder was added thereto (the molar ratio of spermidine to sodium hyaluronate was 0.05, the concentration of HA was 20 mg/mL), stirred until the powder was completely dissolved, and the dissolution time was 3 h.
(2) Then, 3.56 g EDC (the molar ratio of EDC to sodium hyaluronate was 1.5) and 0.713 g NHS were weighed and added into the aforementioned mixed solution, stirred at room temperature for 40 min, and then the system was placed in a 40°C environment for a cross-linking reaction for 3 h, so as to prepare a network cross-linked gel.
(3) 0.68 g of the spermidine solution was weighed and dispersed into 250 mL of purified water, 5 g of sodium hyaluronate powder was added thereto (the molar ratio of spermidine to sodium hyaluronate was 0.03, the concentration of HA was 20 mg/mL), stirred until the powder was completely dissolved, and the dissolution time was 3 h.
(4) The mixed solution of step (3) was added to the network cross-linked gel of step (2), stirred for 10 min, and then crushed for 3 min with a homogenizer at 10000 rpm with 5 min intervals, and repeated 5 times. Then, 0.213 g EDC (the molar ratio of EDC to sodium hyaluronate was 0.09) and 0.043 g NHS were added into the aforementioned system, and the system was further stirred at room temperature for 40 min, and then the system was placed in a 40°C environment for a cross-linking reaction for 16 h to prepare an interlocking cross-linked gel.

The interlocking cross-linked gel was cut with scissors into small pieces of 0.5 cm equivalent diameter and filled into dialysis bags, the dialysis bag was sealed at both ends with a clamp and placed in 9 L phosphate solution containing 2 g sodium hydroxide (the molar ratio of hydroxide to sodium hyaluronate was 2) for dialysis, the dialysis temperature was 40°C, and after 2 h, the solution was changed into a 9 L neutral phosphate solution for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(5) The gel having a concentration C was diluted and compounded until the concentration of sodium hyaluronate was 15 mg/mL, lidocaine hydrochloride was added, using a neutral dialysate as a diluent, and the content of lidocaine hydrochloride in the gel was 3 mg/mL. The compounded gel was crushed, filled into a pre-filled syringe, and subjected to moist heat sterilization at 121°C for 15 min to obtain an injectable interlocking cross-linked sodium hyaluronate hydrogel.

### Example 4 Preparation of an injectable interlocking cross-linked sodium hyaluronate hydrogel

(1) An 8% aqueous solution of spermidine having a pH = 6.0 was prepared and stored refrigerated for use. 0.68 g of the solution was weighed and dispersed into 250 mL of purified water, 5 g of sodium hyaluronate powder was added thereto (the molar ratio of spermidine to sodium hyaluronate was 0.03, the concentration of HA was 20 mg/mL), stirred until the powder was completely dissolved, and the dissolution time was 3 h.
(2) 1.422 g of EDC (the molar ratio of EDC to sodium hyaluronate was 0.6) and 0.285 g of NHS were weighed and dispersed the aforementioned mixed solution, stirred at room temperature for 40 min, and then the system was placed in a 40°C environment for a cross-linking reaction for 3 h, so as to prepare a network cross-linked gel.
(3) 1.13 g of the spermidine solution was weighed and dispersed into 250 mL of purified water, 5 g of sodium hyaluronate powder was added thereto (the molar ratio of spermidine to sodium hyaluronate was 0.05, the concentration of HA was 20 mg/mL), stirred until the powder was completely dissolved, and the dissolution time was 3 h.
(4) The mixed solution of step (3) was added to the network cross-linked gel of step (2), stirred for 10 min, and then crushed for 3 min with a homogenizer at 10000 rpm with 5 min intervals, and repeated 5 times. Then, 0.355 g EDC (the molar ratio of EDC to sodium hyaluronate was 0.15) and 0.071 g NHS were added into the aforementioned system, and the system was further stirred at room temperature for 40 min, and then the system was placed in a 40°C environment for a cross-linking reaction for 16 h, so as to prepare an interlocking cross-linked gel.

The interlocking cross-linked gel was cut with scissors into small pieces with an equivalent diameter of 0.5 cm and filled into dialysis bags, the dialysis bag was sealed at both ends with a clamp and placed in 9 L phosphate solution containing 2 g sodium hydroxide (the molar ratio of hydroxide to sodium hyaluronate was 2) for dialysis, the dialysis temperature was 40°C, and after being changed to a 9 L neutral phosphate solution was used for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(5) The gel having a concentration C was diluted and compounded until the concentration of sodium hyaluronate was 15 mg/mL, lidocaine hydrochloride was added, using a neutral dialysate as a diluent, and the content of lidocaine hydrochloride in the gel was 3 mg/mL. The compounded gel was crushed, filled into a pre-filled syringe, and subjected to moist heat sterilization at 121°C for 15 min to obtain an injectable interlocking cross-linked sodium hyaluronate hydrogel.

### Example 5 Preparation of an injectable interlocking cross-linked sodium hyaluronate hydrogel

(1) An 8% aqueous solution of spermine with pH = 6.0 was prepared and stored refrigerated for use. 0.94 g of the solution was weighed and dispersed into 250 mL of purified water, 5 g of sodium hyaluronate powder was added thereto (the molar ratio of the spermine to sodium hyaluronate was 0.03, and the concentration of HA was 20 mg/mL), stirred until the powder was completely dissolved, and the dissolution time was 3 h.
(2) 4.74 g of EDC (the molar ratio of EDC to sodium hyaluronate was 2.00) and 0.95 g of NHS were weighed and added into the aforementioned mixed solution, stirred at room temperature for 40 min, and then the system was placed in a 40°C environment for a cross-linking reaction for 3 h, so as to prepare a network cross-linked gel.
(3) 1.88 g of the spermine solution was weighed and dispersed into 250 mL of purified water, 5 g of sodium hyaluronate powder was added thereto (the molar ratio of spermine to sodium hyaluronate was 0.06, and the concentration of HA was 20 mg/mL), stirred until the powder was completely dissolved, the dissolution time was 3 h.
(4) The mixed solution of step (3) was added to the network cross-linked gel of step (2), stirred for 10 min, and then crushed for 3 min with a homogenizer at 10000 rpm with 5 min intervals, and repeated 5 times. Then, 0.427 g EDC (the molar ratio of EDC to sodium hyaluronate was 0.18) and 0.086 g NHS were added into the aforementioned system, and the system was further stirred at room temperature for 40 min, and then the system was placed in a 40°C environment for a cross-linking reaction for 16 h, so as to prepare an interlocking cross-linked gel.

The interlocking cross-linked gel was cut with scissors into small pieces of 0.5 cm equivalent diameter and filled into dialysis bags, the dialysis bag was sealed at both ends with a clamp and placed in 9 L phosphate solution containing 4 g of sodium hydroxide (the molar ratio of hydroxide to sodium hyaluronate was 8), the dialysis temperature was 40°C, and after 2 h, the solution was changed into a 9 L neutral phosphate solution for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(5) The gel having a concentration C was diluted and compounded until the concentration of sodium hyaluronate was 15 mg/mL, lidocaine hydrochloride was added, using a neutral dialysate as a diluent, and the content of lidocaine hydrochloride in the gel was 3 mg/mL. The compounded gel was crushed, filled into a pre-filled syringe, and subjected to moist heat sterilization at 121°C for 15 min to obtain an injectable interlocking cross-linked sodium hyaluronate hydrogel.

### Comparative Example 1 Preparation of cross-linked sodium hyaluronate hydrogel

(1) An 8% aqueous solution of spermidine having a pH = 6.0 was prepared and stored refrigerated for use. 1.13 g of the solution was weighed and dispersed into 250 mL of purified water, 5 g of sodium hyaluronate powder was added thereto (the molar ratio of spermidine to sodium hyaluronate was 0.05, the concentration of HA was 20 mg/mL), stirred until the powder was completely dissolved, and the dissolution time was 3 h. Then, 0.356 g of EDC (the molar ratio of EDC to sodium hyaluronate was 0.15) and 0.071 g of NHS were added into the aforementioned mixed solution, stirred at room temperature for 40 min, and then the system was placed in an environment of 40°C and cross-linked for 3 h to prepare a single cross-linking degree gel (component I).

In addition, 0.68 g of the spermidine solution was weighed and dispersed into 250 mL of purified water, 5 g of sodium hyaluronate powder was added thereto (the molar ratio of spermidine to sodium hyaluronate was 0.03, the concentration of HA was 20 mg/mL), stirred to make it completely soluble, and the dissolution time was 3 h. Then, 0.213 g EDC (the molar ratio of EDC to sodium hyaluronate was 0.09) and 0.043 g NHS were added into the aforementioned sodium hyaluronate gel system , and the system was stirred at room temperature for 40 min and then subjected to cross-linking reaction in an environment of 40°C for 3 h, so as to prepare a single cross-linking degree gel (component II).

(2) The component I and component II gels were mixed in the same beaker at a mass ratio of 1:1, and the gel was cut into pieces with an equivalent diameter of 0.5 cm by scissors, the gel was placed in a dialysis bag, both ends of the dialysis bag were sealed by a clamp, and the dialysis bag was placed in 9 L phosphate solution containing 2 g of sodium hydroxide (the molar ratio of hydroxide to sodium hyaluronate was 2), the dialysis temperature was 40°C, and after 2 h, the solution was changed to 9 L of a neutral phosphate solution for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(3) The gel having a concentration C was diluted and compounded until the concentration of sodium hyaluronate was 15 mg/mL, adding lidocaine hydrochloride, using a neutral dialysate as the diluent, and the content of lidocaine hydrochloride in the gel being 3 mg/mL. The compounded cross-linked gel was crushed, filled into a pre-filled syringe, and subjected to moist heat sterilization at 121°C for 15 min, so as to obtain a cross-linked sodium hyaluronate hydrogel.

### Comparative Example 2 Preparation of cross-linked sodium hyaluronate hydrogel

An interlocking cross-linked gel was prepared according to the process parameters in Example 2, except that no alkaline dialysis was used. In the dialysis procedure, only 9 L of neutral phosphate solution was used for dialysis, the dialysis temperature was 25°C, the solution was changed every 3 h, and the dialysis was performed overnight for 20 h. The compounded gel was crushed, filled into a pre-filled syringe, and subjected to moist heat sterilization at 121°C for 15 min, so as to finally obtain a cross-linked sodium hyaluronate hydrogel.

### Comparative Example 3 Preparation of cross-linked sodium hyaluronate hydrogel

(1) An 8% aqueous solution of spermidine having a pH = 6.0 was prepared and stored refrigerated for use. 2.25 g of the solution was weighed and dispersed into 500 mL of purified water, 10 g of sodium hyaluronate powder was added thereto (the molar ratio of spermidine to sodium hyaluronate was 0.05, the concentration of HA was 20 mg/mL), stirred until the powder was completely dissolved, and the dissolution time was 3 h. Then, 0.711 g EDC (the molar ratio of EDC to sodium hyaluronate was 0.15) and 0.143 g NHS were added into the above mixed solution, stirred at room temperature for 40 min, and then the system was placed in a 40°C environment for a cross-linking reaction for 16 h to prepare a single cross-linking degree gel.
(2) The gel was cut into small pieces with an equivalent diameter of 0.5 cm with scissors, placed into a dialysis bag, and both ends of the dialysis bag were sealed with a clamp, dialysis was carried out in 9 L of phosphate solution containing 2 g of sodium hydroxide (the molar ratio of hydroxide to sodium hyaluronate was 2), the dialysis temperature was 40°C, after 2 h, the solution was changed to 9 L of a neutral phosphate solution for dialysis, the dialysis temperature was 25°C, the solution was changed every 3 h, and was subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$
(3) The gel having a concentration C was diluted and compounded until the concentration of sodium hyaluronate was 15 mg/mL, lidocaine hydrochloride was added, using a neutral dialysate as the diluent, and the content of lidocaine hydrochloride in the gel was 3 mg/mL. The compounded cross-linked gel was crushed, filled into a pre-filled syringe, and subjected to moist heat sterilization at 121°C for 15 min, so as to obtain a cross-linked sodium hyaluronate hydrogel.

### Comparative Example 4 Preparation of cross-linked sodium hyaluronate gel

(1) An 8% aqueous solution of spermidine having a pH = 6.0 was prepared and stored refrigerated for use. 0.68 g of the solution was weighed and dispersed into 250 mL of purified water, 5 g of sodium hyaluronate powder was added thereto (the molar ratio of spermidine to sodium hyaluronate was 0.03, the concentration of HA was 20 mg/mL), stirred until the powder was completely dissolved, and the dissolution time was 3 h.
(2) 0.711 g EDC (the molar ratio of EDC to sodium hyaluronate was 0.3) and 0.143 g NHS were weighed and added to the aforementioned mixed solution, stirred same at room temperature for 40 min, and then subjecting the system to an environment of 40°C for a cross-linking reaction for 3 h, so as to prepare a network cross-linked gel.
(3) 1.13 g of the spermidine solution was weighed and dispersed into 250 mL of purified water, 5 g of sodium hyaluronate powder was added thereto (the molar ratio of spermidine to sodium hyaluronate was 0.05, the concentration of HA was 20 mg/mL), stirred until the powder was completely dissolved, and the dissolution time was 3 h.
(4) The mixed solution of step (3) was added to the network cross-linked gel of step (2), stirred for 10 min, and then crushed for 3 min with a homogenizer at 10000 rpm with 5 min intervals, and repeated 5 times. Then, 0.355 g EDC (the molar ratio of EDC to sodium hyaluronate was 0.15) and 0.071 g NHS were added into the aforementioned system, and the system was further stirred at room temperature for 40 min, and then the system was subjected to cross-linking reaction in a 40°C environment for 16 h to prepare a cross-linked gel.

The cross-linked gel was cut into small blocks with an equivalent diameter of 0.5 cm, placed into a dialysis bag, and both ends of the dialysis bag were sealed with a clamp, dialysis was carried out in 9 L of phosphate solution containing 2 g of sodium hydroxide (the molar ratio of hydroxide to sodium hyaluronate was 2), the dialysis temperature was 40°C, and after 2 h, the solution was changed to a 9 L neutral phosphate solution for dialysis, in which the dialysis temperature was 25°C, the solution was changed every 3 h and subjected to overnight dialysis for 20 h. The mass of a blank dialysis bag (M₀) and a dialysis bag after loading (M₁) were accurately weighed before dialysis; the total mass of the dialysis bag and the gel (M₂) was accurately weighed after dialysis; the initial HA concentration (N) was 20 mg/mL; and the sodium hyaluronate content (C) of the gel after dialysis was calculated: $C = \frac{N \times \left({M}_{1}-{M}_{0}\right)}{{M}_{2} - {M}_{0}}$

(5) The gel having a concentration C was diluted and compounded until the concentration of sodium hyaluronate was 15 mg/mL, lidocaine hydrochloride was added, using a neutral dialysate as a diluent, and the content of lidocaine hydrochloride in the gel was 3 mg/mL. The compounded gel was crushed, filled into a pre-filled syringe, and subjected to moist heat sterilization at 121°C for 15 min, so as to obtain a cross-linked sodium hyaluronate hydrogel.

### Performance test 1 Elastic Modulus Test

The modulus of elasticity of the hydrogel samples before and after sterilization of Examples 1-5, Comparative Examples 1-4 was measured using a rheometer.

**Table 1 Elastic Modulus of Samples Before and After Sterilization**

| Sample | Elastic modulus before sterilization (Pa) | Elastic modulus after sterilization (Pa) | Elastic modulus loss rate (%) |
|---|---|---|---|
| Example 1 | 326 | 301 | 7.5 |
| Example 2 | 350 | 329 | 6 |
| Example 3 | 309 | 277 | 10.1 |
| Example 4 | 263 | 232 | 11.7 |
| Example 5 | 391 | 364 | 7 |
| Comparative Example 1 | 246 | 201 | 24 |
| Comparative Example 2 | untested^{note} | 401 | - |
| Comparative Example 3 | 228 | 183 | 20 |
| Comparative Example 4 | 248 | 208 | 16 |

| | | | |
|---|---|---|---|
| Note: The sample exhibited phase separation "water-gel separation" before sterilization and could not be tested. | | | |

It can be seen from Table 1 that the hydrogel samples of the examples have high elastic modulus before and after sterilization, and the loss rate of elastic modulus was almost within 12%, exhibiting good resistance to moist heat sterilization, and all satisfy clinical use requirements.

The hydrogel sample of Example 1 has a high elastic modulus before and after sterilization, a loss rate of only 7.5%, and exhibits high supporting properties and stability. This is because the carboxyl group in the HA molecule forms an ester bond with the activated hydroxyl group at the same time when cross-linking is performed to form a network structure in the first step, which exerts a occupancy effect; then, the added HA and the cross-linking agent can uniformly interpenetrate into the network structure formed in the first step, and then perform secondary cross-linking in the presence of the catalyst, so as to obtain a cross-linked gel having an interlocking structure. Further, in the process of alkaline dialysis, the released HA can further enhance the intermolecular force and binding force of the cross-linked network, so that the gel product has a high elastic modulus, good supporting properties, and strong resistance to moist heat sterilization.

The modulus of elasticity of the hydrogel sample of Example 2 is higher than that of Example 1, with a lower loss rate of only 6%. This is because in the first step, the cross-linking reaction has a low degree of cross-linking, and the formed cross-linking network has a loose structure, which is more conducive to the subsequent interpenetration and intertwining of the HA molecules, so that the networks with different layers are more conducive to the formation of a tight interlocking structure, and therefore exhibit better supporting properties and stronger resistance to moist heat sterilization.

The elastic modulus of the hydrogel sample of Example 3 is slightly lower than that of Example 1 because, although an interlocking structure can be formed through a two-step reaction, in the case where the overall degree of cross-linking is similar, the amount of the cross-linking agent used in the first step cross-linking reaction is relatively large, and the degree of cross-linking was relatively high, the formed cross-linked network structure is relatively dense, and the interpenetration capability of HA molecules is relatively weak, and therefore, the intermolecular force is slightly reduced, the samples exhibit a decrease in elastic modulus.

The elastic modulus of the hydrogel sample of Example 2 is higher than that of Example 4, this is because in the first step cross-linking reaction, the amount of the catalyst of Example 2 is relatively high, enabling complete occupation of HA; therefore, the interlocking structure of the gel product is relatively large, the network structure is denser, and the gel supporting properties are better; however, the amount of the catalyst added in Example 4 is slightly low, so that the carboxyl site of HA is not fully occupied, thereby reducing the proportion of the interlocking structure in the cross-linked gel, and slightly reducing the support performance of the gel. This also illustrates that in the first step cross-linking the best mode is "complete occupation".

In addition, it can be seen from the data in Example 5 that the use of spermine as a cross-linking agent can also achieve a good cross-linking effect, form an interlocking cross-linked hydrogel of hyaluronan or salt thereof, and both the supporting properties and the moist heat sterilization resistance satisfy clinical use requirements.

It can be seen from Example 3 and Comparative Example 1 that the elastic modulus of the hydrogel sample of Comparative Example 1 is low before and after sterilization, at the same time, the loss rate of the elastic modulus of the sample of Comparative Example 1 is significantly different, up to 24%, from the sample of Example 3, the main reason is that Comparative Example 1 is a common cross-linked gel prepared by physical mixing rather than the interlocking cross-linked gel prepared by two-step chemical cross-linking, the acting force of the physically mixed two gel structures is small, and therefore the elastic modulus of the gel product is low, and the capability of resisting moist heat sterilization is also weak.

Comparing Example 2 and Comparative Example 2, the latter exhibited phase separation ("water-gel separation") before sterilization, making modulus measurement impossible. This is because the EDC/NHS catalytic system inevitably generates ester bonds, which can induce supersaturation in the gel. Since Comparative Example 2 employed only neutral dialysis (without alkaline dialysis), these ester bonds were not effectively broken. Consequently, at a low HA concentration (15 mg/mL), the sample underwent phase separation, failing to meet clinical requirements. In contrast, Example 2 utilized appropriate alkaline dialysis to hydrolyze the majority of ester bonds while preserving HA glycosidic bonds. This not only improved sterilization resistance but also resolved the phase separation issue at the given HA concentration.

At the same time, it can be seen from the post-sterilization elastic modulus data that the cross-linking degree of the gel products of the two products is the same; however, the elastic modulus (329 Pa) of Example 2 is slightly lower than the elastic modulus (401 Pa) of Comparative Example 2, because the elastic modulus is higher in that some ester bonds are still retained in the gel even after the moist heat sterilization without alkali dialysis of Comparative Example 2. However, the ester bond is extremely unstable, which adversely affects the storage stability of the product and also restricts the viscosity of the gel product (see performance test 3).

It can be seen from the data of Example 2 and Comparative Example 3 that compared with a common one-step cross-linked gel, the interlocking structure design of Example 2 brings higher supporting properties to the product, and is more beneficial to the tissue repair of the shaped part; however, with regard to the hydrogel sample of Comparative Example 3 which is one-step cross-linked, merely increasing the degree of cross-linking would lead to an excessive extrusion force of the hydrogel sample, and therefore the clinical use is limited (see performance test 5).

It can be seen from the data of Example 2 and Comparative Example 4 that during the first step of the cross-linking reaction, the amount of EDC/NHS added is too low, and at this time, the carboxyl sites of HA not participating in the cross-linking reaction are not occupied by EDC/NHS, so that free HA consumes the cross-linking agent added in the second step, thereby reducing the efficiency of the cross-linking reaction of HA interpenetrating the first network, reducing the formation of an interlocking structure, and further causing the supporting property of the hydrogel sample to be significantly reduced.

### Performance test 2 Esterase degradation experiment

A 100 U/mL esterase solution (carboxylesterase, purchased from Sigma) was prepared using purified water for use. 0.618 g of boric acid and 0.01 g of sodium hydroxide were weighed and dissolved in 1 L of purified water to obtain a buffer solution with pH=8.65 for use. 2 mL each of the hydrogel sample of Example 2 and the hydrogel sample of Comparative Example 2 were respectively taken, and 300 µL of a stock solution of esterase and 700 µL of a buffer solution of boric acid were added thereto; after being mixed uniformly, an enzymatic hydrolysis curve was recorded.

A rheometer was used with the following procedure to test the change in elastic modulus of the samples over degradation time: testing for 7200 seconds using the rheometer in the Oscillation Time mode at 37°C; Strain:1%; frequency: 0.9Hz. The degradation rates were investigated at 10 min, 30 min, 60 min, 90 min, 120 min. The results are shown in Tables 2 and 3.

**Table 2 Degradation rate of samples in the presence of esterase before sterilization**

| degradation time (min) | Example 2 | Comparative Example 2 |
|---|---|---|
| 10 | 1.1% | 7.8% |
| 30 | 2.0% | 16% |
| 60 | 4.1% | 35% |
| 90 | 5.7% | 57% |
| 120 | 6.2% | 60% |

**Table 3 Degradation rate of samples in the presence of esterase after sterilization**

| degradation time (min) | Example 2 | Comparative Example 2 |
|---|---|---|
| 10 | 0.2% | 1.8% |
| 30 | 0.6% | 6% |
| 60 | 1.0% | 10% |
| 90 | 1.1% | 14% |
| 120 | 1.1% | 15% |

It can be seen from the pre-sterilization data in table 2 that compared with Comparative Example 2, the degradation rate of the hydrogel sample before sterilization in Example 2 is obviously lower than the degradation rate of the hydrogel sample in Comparative Example 2, and the degradation rate is only 6.2% after 120 min of enzymatic hydrolysis treatment, this is because in Example 2, the majority of ester bonds generated in the cross-linking process are eliminated by alkaline dialysis, and therefore the substrate of the esterase is less; the hydrogel sample of Comparative Example 2 which has not been treated by alkaline dialysis retains a large number of ester bonds, and in the presence of esterase, the degradation rate of hydrogel is high, even up to 60%.

It can be seen from the post-sterilization data in Table 3 that the degradation rates of the hydrogel samples in Example 2 and the hydrogel samples in Comparative Example 2 are both reduced because the ester bonds are destroyed during moist heat sterilization. However, the degradation rate of the hydrogel sample of Comparative Example 2 is still obviously higher than that of Example 2, because the unstable ester bonds originally exist in Comparative Example 2, and the number of ester bonds is large. Although the ester bonds will be destroyed by moist heat sterilization, the ester bonds retained in the final product are relatively more, and the degradation rate after esterase treatment for 120 min is 15%, which is obviously higher than the degradation rate of the sample of Example 2 (1.1%). Further, after 60 min of esterase treatment, the degradation rate of the hydrogel sample of Example 2 remains almost unchanged; however, for the hydrogel sample of Comparative Example 2, with the increase of the esterase time, the degradation rate is still gradually increased, which also indicates that the existence of ester bonds will affect the storage stability of the product, and at the same time, due to the large number of unstable ester bonds, the modulus would be greatly reduced during storage, and the clinical use of the product will also be affected. Therefore, the unstable ester bond produced in the cross-linking process can be eliminated by suitable alkaline dialysis in the present invention, which is beneficial to improving the storage stability of the product.

### Performance Test 3 Viscosity

### (1) Drop Weight Method

The hydrogel samples of Examples 1-4 and Comparative Examples 1-4 were filled into 1 mL pre-filled syringes (with air bubbles removed), a chlorinated butyl rubber piston, a push rod and a booster were mounted, a 27G needle was then mounted, and the syringes were clamped by an appropriate clamp, activating the motor to move the upper and lower clamps to an appropriate position, pushing the push rod at a constant speed of 30 mm/min, when a constant force was obtained, the gel was extruded from a needle to a drop detachment, and was a sample; a total of 10 samples were collected and weighed, and the average weight of the samples was calculated, the larger the weight, the better the viscosity. The results are shown in Table 4.

**Table 4 Viscosity characterization**

| Sample | Droplet mass (mg) |
|---|---|
| Example 1 | 25 |
| Example 2 | 29 |
| Example 3 | 23 |
| Example 4 | 21 |
| Comparative Example 1 | 18 |
| Comparative Example 2 | 15 |
| Comparative Example 3 | 16 |
| Comparative Example 4 | 19 |

The results of Table 4 show that the interlocking cross-linked hydrogels prepared by two-step chemical cross-linking (Examples 1-4) have higher viscosity than the hydrogels physically mixed using only gels of different degrees of cross-linking (Comparative Example 1). This is because, when a cross-linking reaction is performed in two steps, sodium hyaluronate and a cross-linking agent molecule form interlocked molecular chain loops by means of the cross-linking reaction, and three-dimensional network structures penetrate each other, so that the intermolecular binding forces of HA and the molecular tension are both greatly improved, and the cross-linked HA network structure having an interlocking structure is more compact, thereby exhibiting excellent viscosity.

Compared with Example 2, in the hydrogel sample of Comparative Example 4, since no effective space-occupying ester bond is formed in the first step cross-linking process, the efficiency of HA cross-linking is reduced, and it is difficult to release a linear HA chain after dialysis; therefore, the intermolecular forces between the cross-linked HA network structures is weak, and therefore the viscosity of the hydrogel sample of Comparative Example 4 is low.

### (2) Appearance

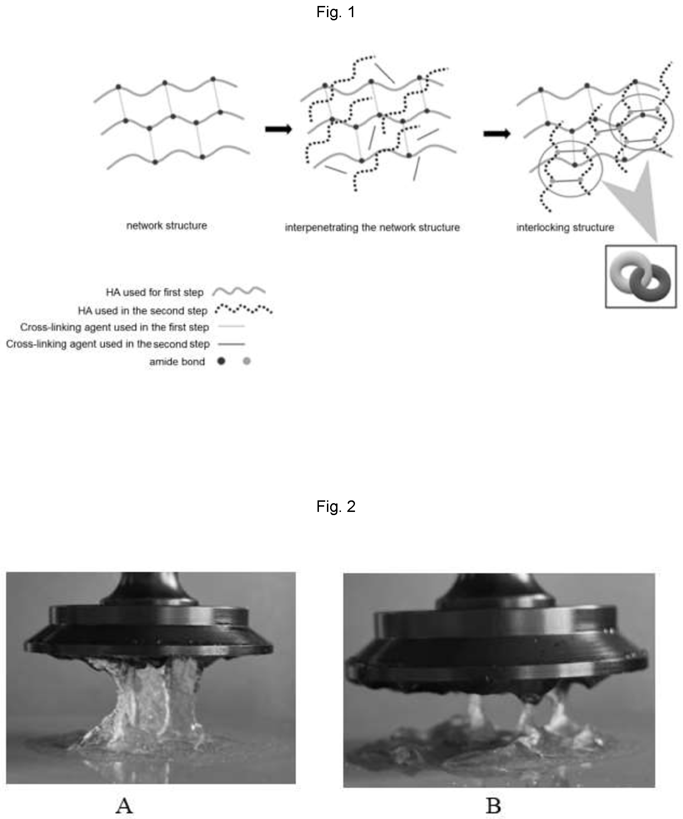

The appearance of the samples of Example 2 and Comparative Example 1 from step (1) is observed and the results are shown in Fig. 2. As can be seen from Fig.2, it is further shown from the appearance of the product that the sample of the interlocking cross-linked hydrogel prepared in Example 2 (A in Fig.2) has higher viscosity than the hydrogel sample of Comparative Example 1 (B in Fig.2). Higher viscosity is crucial for the clinical use of the product, and shows that the product has good ductility after injection, is easy to spread and plastic, has a strong binding capacity with a tissue, and is not displaced, so that the injection filling effect is more natural, and the maintenance time is longer.

### Performance test 4 in vitro degradation

1.0 mL of hyaluronidase solution at 30 U/mL (the hyaluronidase was purchased from Sigma-Aldrich) was added to 2.0 mL of the hydrogel samples of Examples 1-4 and Comparative Examples 1-4, and the hydrogel samples and the enzyme solution were stirred and mixed manually (ensuring uniform mixing of the hydrogel and enzyme solution). Then, the modulus of elasticity of the hydrogel samples within 2 h was measured by using a rheometer (prior to loading, the temperature of the Peltier plate was first equilibrated to 4°C), and the rate of enzyme digestion was represented by a variation value of the modulus of elasticity. The results are shown in Table 5.

**Table 5 Degradation Rate (%)**

| Degradation time | Example 1 | Example 2 | Example 3 | Example 4 | Comparative Example 1 | Comparative Example 2 | Comparative Example 3 | Comparative Example 4 |
|---|---|---|---|---|---|---|---|---|
| 10 min | 7 | 3 | 9 | 11 | 12 | 18 | 19 | 9 |
| 30 min | 18 | 12 | 22 | 26 | 28 | 40 | 36 | 25 |
| 90 min | 58 | 45 | 61 | 68 | 72 | 56 | 80 | 57 |
| 120 min | 66 | 56 | 70 | 78 | 85 | 70 | 95 | 86 |

The above in vitro degradation data simulating in vivo conditions show that after 120 min of degradation, the enzymatic degradation resistance of the hydrogel samples of Examples 1-4 is significantly higher than that of Comparative Examples 1, 3 and 4, indicating that the interlocking structure can significantly improve the retention time of the product in vivo after injection, and fully achieve its long-lasting filling effect. At the same time, comparing the degradation rates of Example 2 and Comparative Example 4, it can be seen that in the preparation process of the sample of Comparative Example 4, no EDC/NHS space-occupying step is employed in the first-stage cross-linking, which results in a low cross-linking efficiency in the second step, almost no interlocking structure is formed, and at the same time, the intermolecular force of the interlocking network structure cannot be further enhanced by using the HA chain formed after the alkaline dialysis, and thus the resistance to enzymatic degradation is relatively weak.

The absence of alkaline dialysis treatment in Comparative Example 2 has little effect on the enzymatic degradation resistance, and therefore the degradation rate is slightly lower than that of Example 2, but has a significant impact on the viscosity and stability of the product (Performance tests 2 and 3).

### Performance test 5 Extrusion force

The hydrogel samples of Examples 1-5 and Comparative Examples 1-4 were taken separately, and the extrusion force of each sample was tested, and the results are shown in Table 6.

**Table 6 Extrusion force**

| Sample | Extrusion force (N) |
|---|---|
| Example 1 | 17 |
| Example 2 | 14 |
| Example 3 | 16 |
| Example 4 | 17 |
| Example 5 | 15 |
| Comparative Example 1 | 18 |
| Comparative Example 2 | 25 |
| Comparative Example 3 | 20 |
| Comparative Example 4 | 21 |

| | |
|---|---|
| Note: (1) Extrusion force Test Method Reference YY/T 0962-2021 Method; (2) the injection needle used for testing was 27G×1/2. | |

It can be seen from Table 6 that the extrusion force of the hydrogel sample of Examples 1-5 was relatively small, which facilitates the clinical injection. The extrusion force of the hydrogel sample of Example 2 was smaller than that of the hydrogel sample of Comparative Example 2, because alkaline dialysis released a linear structure of sodium hyaluronate when the ester bond was broken, and the effect was equivalent to the adding free HA, which achieves the lubrication effect, so that the product can be injected more easily.

At the same time, the extrusion force of the hydrogel sample of Example 2 was less than that of the hydrogel sample of Comparative Example 3, because, compared with hydrogels prepared by one-step chemical cross-linking, hydrogels prepared by two-step chemical cross-linking form spatial structures with different hierarchies, so that released free HA molecules interpenetrate more completely, thus, the spatial structure becomes more compact, enhancing the product's supportiveness while maintaining ease of injection, thereby meeting clinical injection requirements. By the same reasoning, in Comparative Example 4, since the pre-cross-linking did not form the HA occupying effect, the free HA has already participated in the cross-linking reaction and the HA interpenetrated within the network is difficult to release, and therefore, the extrusion force was also high.

The foregoing descriptions are merely Examples of the present invention, and are not intended to limit the patent scope of the present invention. Any equivalent structural change made by using the content of the description of the present invention, or direct or indirect application in other related technical fields, shall fall within the scope of patent protection of the present invention.

## Claims

1. An injectable interlocking cross-linked hydrogel of hyaluronan or salt thereof, wherein the hydrogel is obtained by a two-step cross-linking reaction, firstly performing a first step cross-linking reaction on hyaluronan or salt thereof with a cross-linking agent to form a network structure, and subsequently interpenetrating the hyaluronan or salt thereof and the cross-linking agent into the network structure, performing a second step cross-linking reaction to obtain an injectable interlocking cross-linked hydrogel of hyaluronan or salt thereof.

2. The injectable interlocking cross-linked hydrogel of hyaluronan or salt thereof according to claim 1, wherein the cross-linking agent is an endogenous polyamine comprising spermidine, spermine and derivatives thereof;
the hyaluronan or salt thereof is selected from one or more of sodium hyaluronate, zinc hyaluronate, potassium hyaluronate and calcium hyaluronate;
the hyaluronan or salt thereof has a molecular weight of 500-2600KDa.

3. The injectable interlocking cross-linked hydrogel of hyaluronan or salt thereof according to claim 2, wherein the hyaluronan or salt thereof is sodium hyaluronate.

4. The injectable interlocking cross-linked hydrogel of hyaluronan or salt thereof according to any one of claims 1-3, wherein the particle size of the injectable interlocking cross-linked hydrogel of hyaluronan or salt thereof is 50-650 µm;
in the injectable interlocking cross-linked hydrogel of hyaluronan or salt thereof, the concentration of hyaluronan or salt thereof is 10 -25 mg/mL.

5. A preparation method for an injectable interlocking cross-linked hydrogel of hyaluronan or salt thereof, wherein the preparation method comprises the following steps:
(1) mixing a cross-linking agent with hyaluronan or salt thereof to prepare a mixed solution A of the cross-linking agent and the hyaluronan or salt thereof;
(2) adding a catalyst to the mixed solution A of the cross-linking agent and hyaluronan or salt thereof, and performing a first step cross-linking reaction, so as to prepare a cross-linked gel having a network structure;
(3) mixing the cross-linking agent with the hyaluronan or salt thereof to prepare a mixed solution B of the cross-linking agent and the hyaluronan or salt thereof;
(4) adding the mixed solution B prepared in step (3) to the cross-linked gel having a network structure prepared in step (2), performing a second cross-linking reaction in the presence of a catalyst to form a cross-linked gel having an interlocking structure, and performing dialysis.

6. The preparation method according to claim 5, wherein in steps (1) and (3), the cross-linking agent is an endogenous polyamine comprising spermidine, spermine and derivatives thereof; and
in steps (1) and (3), the hyaluronan or salt thereof is selected from one or more of sodium hyaluronate, zinc hyaluronate, potassium hyaluronate and calcium hyaluronate.

7. The preparation method according to claim 5, wherein the mass percentage of the hyaluronan or salt thereof in the mixed solution A and the mixed solution B is 1%-5%.

8. The preparation method according to claim 5, wherein the molar ratio of the cross-linking agent to the hyaluronan or salt thereof in the mixed solution A and the mixed solution B is 0.01-0.1.

9. The preparation method according to claim 8, wherein the molar ratio of the cross-linking agent to the hyaluronan or salt thereof in step (1) is lower than the molar ratio of the cross-linking agent to the hyaluronan or salt thereof in step (3).

10. The preparation method according to claim 5, wherein in steps (2) and (4), the catalyst is selected from one or more of a carbodiimide, a bromophosphonium salt formed from triphenylphosphine and a bromide, a carbonium salt, and 4-(4,6-dimethoxytriazin-2-yl)-4-methylmorpholine hydrochloride (DMTMM).

11. The preparation method according to claim 10, wherein in step (2), the molar ratio of the catalyst to the hyaluronan or salt thereof in the mixed solution A is 0.6-3, and the cross-linking time is 1-3 h; and/or
in step (4), the molar ratio of the catalyst to the hyaluronan or salt thereof in the mixed solution B is 0.03-0.3, and the cross-linking time is 1-24 h.

12. The preparation method according to claim 5, wherein in the dialysis of step (4), firstly using an alkaline dialysate for dialysis, and then using a neutral dialysate for dialysis;
in the alkaline dialysate, the molar ratio of hydroxide to total hyaluronan or salt thereof used in steps (1) and (3) is 1:1-10:1, the alkaline dialysis temperature is 10-40°C, and the alkaline dialysis time is 1-10 h; and/or
the neutral dialysis temperature is 10-40°C and the neutral dialysis time is 1-20 h.

13. Use of the injectable interlocking cross-linked hydrogel of hyaluronan or salt thereof of any one of claims 1-4 or the injectable interlocking cross-linked hydrogel of hyaluronan or salt thereof obtained according to the preparation method of any one of claims 5-12 in the preparation of a non-therapeutic tissue filling and repairing material or a pharmaceutical carrier.
